# EUROPEAN PATENT APPLICATION

(11) **EP 4 302 816 A1**
(43) Date of publication of application: **10.01.2024**
(21) Application number: 21929265.3
(22) Date of filing: 05.03.2021
(51) Int. Cl.: A61M 31/00, A61J 3/07, A61B 5/07, A61B 5/145

(54) **ORAL DRUG DELIVERY DEVICE**

(71) Applicant: Bitherapeutics Inc., Seoul 08646 (KR)
(72) Inventor: CHO, Jin Taeg, Seoul 01799 (KR); JANG, Da In, Hwaseong-si Gyeonggi-do 18444 (KR)
(74) Representative: Caspary, Karsten
(86) International application number: PCT/KR2021/002720
(87) International publication number: WO 2022/186403

(57) **Abstract**

The disclosed invention relates to a swallowable oral drug delivery device comprising: a driving part including an actuator, and a cylinder linearly moving by means of the actuator; a drug accommodation part including a first chamber for accommodating a piston linked with the cylinder to linearly move and an inactivated first drug, a second chamber for accommodating a second drug activating the first drug, a separation membrane for isolating the first chamber from the second chamber, and an outlet membrane for closing an outlet allowing either the first chamber or the second chamber to communicate with an external environment; a sensing part for identifying the movement or position of the drug delivery device; and a control part for receiving a signal provided from the sensing part, and controlling the operation of the actuator on the basis of the signal.

## Description

### [Technical Field]

The present invention relates to a swallowable small oral substance-delivery device, and more particularly, to an oral drug delivery device, which contains any substance, and during oral intake, releases the substance after it is stably delivered to a target organ (e.g., the stomach, small intestine or large intestine) in the digestive tract.

### [Background Art]

The technology for effectively introducing various substances, drugs, microorganisms, etc. (hereinafter referred to as "substances"), which can be expected to have physiological or pharmacological effects on animals (including humans), into a target organ in the animals' body is just as important as developing the substances themselves. For example, if a substance that, upon oral administration, needs to reach the small intestine for its effect to be realized does not properly pass through the stomach properly, it will be difficult to achieve the desired effect. Similarly, if an anticancer drug used for cancer treatment does not reach the cancerous tissue adequately, its therapeutic effect may be minimal.

There are various approaches for effective substance delivery to target organs in a living body. In the case of oral formulations, for example, multiple layers of coating materials can be applied to the surface of the drug to protect the drug until it reaches the target organ. Otherwise, a substance is introduced directly into the target organ in an invasive manner. Recently, a small device that releases a substance has been attached directly or indirectly (e.g., on the skin) to a target organ to deliver the substance periodically for a long time. For example, a micro robot that circulates in the bloodstream has been developed to regularly administer insulin formulations to diabetic patients.

Although a lot of research is required to effectively deliver simple substances or drugs into a living body, it is even more difficult to introduce living microorganisms (e.g., probiotics) into target organs in the living body. Working with living microorganisms is sensitive to contamination, making it difficult to introduce them into the living body in an uncontrolled environment. To overcome these limitations, freeze-dried strains are coated with various substances to create protective layers, forming multilayered structures before being delivered to the intestine in the form of oral formulations. However, this approach faces challenges such as difficulty in accurate positioning, time delay for activation from the freeze-dried state, and competition with existing intestinal microorganisms. In many cases, there is an insufficient population of activated microorganisms to compete effectively, leading to a lack of substantial efficacy.

### [Disclosure]

### [Technical Problem]

An object of the present invention is to provide an oral drug delivery device, which contains a substance having a positive physiological or pharmacological effect on a living body, and during oral intake, releases an activated substance after it is stably delivered to a target organ such as the stomach, small intestine or large intestine in the digestive tract.

### [Technical Solution]

The present invention provides a swallowable oral drug delivery device, comprising: a driving unit including an actuator and a cylinder that is linearly moved by the actuator; a substance-containing unit including: a first chamber, which contains a piston linearly moving in conjunction with the cylinder, and an inactive first substance; a second chamber that contains a second substance for activating the first substance; a separation membrane that isolates the first chamber and the second chamber from each other; and an outlet membrane that closes an outlet allowing either the first chamber or the second chamber to communicate with an external environment; a sensing unit that detects the movement or position of the oral drug delivery device; and a control unit that receives a signal provided from the sensing unit and controls the operation of the actuator based on the received signal.

According to an embodiment, the second substance may be contained in the first chamber, and the first substance may be contained in the second chamber.

In one embodiment of the present invention, the cylinder and the piston may be physically connected to each other, and the sealed state of the substance-containing unit may be maintained at all times. In another embodiment, the cylinder and the piston may be physically separated from each other while each containing a magnetic element, and the poles of each magnetic element may be arranged to exert a repulsive force between the cylinder and the piston.

The first substance may be a freeze-dried microbial strain and the second substance may be a culture medium. In one embodiment of the present invention, the first substance may be anti-Helicobacter pylori probiotics, Lactobacillus bacteria or Bifidobacterium, and the target organ of the oral drug delivery device may be stomach, small intestine or large intestine.

The separation membrane may be ruptured as the piston moves a predetermined distance, causing the first substance and the second substance to be mixed to activate the first substance. In one embodiment of the present invention, the first substance may be is a freeze-dried microbial strain and the second substance may be a culture medium, and the rupture of the separation membrane may occur before oral intake of the oral drug delivery device.

After the first substance is activated or cultured and the oral drug delivery device reaches the target organ, the piston may move a predetermined distance to rupture the outlet membrane, and after the outlet membrane is ruptured, the piston may move according to a predetermined release profile.

The sensing unit may comprise at least one of a dissolved oxygen sensor, a pH sensor, an inertial sensor, and a timer.

In one embodiment of the present invention, the first substance may be a freeze-dried microbial strain and the second substance may be a culture medium, and the target organ of the oral drug delivery device may be a digestive tract. In this case, the control unit may distinguish the large intestine from the stomach and the small intestine based on the concentration of oxygen according to a signal from the dissolved oxygen sensor, distinguish between the stomach and the small intestine based on the acidity according to a signal from the pH sensor, detect the speed and acceleration of the oral drug delivery device according to signals from the inertial sensor and the timer to distinguish between the stomach and the small intestine based on the detected speed and acceleration, and determine whether the time required for the oral drug delivery device to pass through any digestive tract has elapsed after oral intake of the oral drug delivery device according to a signal from the timer.

Moreover, the sensing unit may comprise a plurality of sensors selected from the dissolved oxygen sensor, the pH sensor, the inertial sensor, and the timer, and the control unit may determine whether the oral drug delivery device has reached the target organ by comprehensively evaluating signals from the plurality of sensors.

For example, the control unit may determine whether the oral drug delivery device has reached the target organ by evaluating the signals from the plurality of sensors under an AND condition. Otherwise, the control unit may assign a weight to each of the signals from the plurality of sensors, quantitatively sum up the weighted signals, and if the sum exceeds a predetermined reference value, determine that the oral drug delivery device has reached the large intestine.

The driving unit and the substance-containing unit may be placed in separate bodies and then combined into a single body, which maintains airtightness.

Furthermore, at least the substance-containing unit may preferably be in an oxygen-free environment, for example, in a nitrogen environment.

### [Advantageous Effects]

The oral drug delivery device of the present invention having the above-mentioned configuration exhibits excellent efficiency in substance delivery due to its ability to release an activated substance for an appropriate duration after reaching a target organ.

In particular, for the delivery of probiotics, for example, a small amount of strain in a freeze-dried form and a culture medium for its cultivation are separately provided in the device, and the cultivation is initiated just before oral intake to reach a sufficient population while moving to a target organ, such that living probiotics can be directly released into the target organ. As a result, compared to conventional oral administration methods, this approach provides significantly superior effects in delivering probiotics.

Moreover, since this approach involves cultivating a freeze-dried strain in the drug delivery device and then directly delivering the cultivated strain to the target organ, it is possible to significantly reduce the required amount of strain, minimize potential side effects on the human body, and substantially reduce the production costs.

Furthermore, the oral drug delivery device of the present invention determines whether it has reached the target organ through an incorporated sensor on its own, and thus there is no need to observe or control the introduction process using an external device. That is, the oral drug delivery device of the present invention is a fully self-contained drug delivery device, which, after oral intake, operates autonomously, making it clinically convenient to use and equally user-friendly as conventional oral formulations, and thus its effectiveness is very high.

The effects of the present invention are not limited to those mentioned above, and other effects not mentioned will be clearly understood by those skilled in the art from the following description.

### [Description of Drawings]

FIG. 1 is a view showing the appearance of an oral drug delivery device according to the present invention.
FIG. 2 is a schematic diagram showing the internal configuration of the oral drug delivery device of FIG. 1.
FIG. 3 is a diagram showing a state in which a separation membrane is ruptured (i.e., initiated cultivation) by the movement of a piston in the oral drug delivery device of FIG. 2.
FIG. 4 is a diagram showing a state in which a piston ruptures an outlet membrane to release an activated substance in the oral drug delivery device of FIG. 3.
FIG. 5 is a diagram showing an embodiment of a sensing unit.
FIG. 6 is a diagram schematically showing an example of transit time for each digestive tract and an example of releasing a substance after the oral drug delivery device reaches the ascending colon of the large intestine.

### [Mode for Carrying Out the Invention]

As the present invention allows for various changes and numerous embodiments, particular embodiments will be illustrated in the drawings and described in detail in the written description. However, this is not intended to limit the present invention to particular modes of practice, and it is to be appreciated that all changes, equivalents, and substitutes that do not depart from the spirit and technical scope of the present invention are encompassed in the present invention.

The terms as used herein are provided to merely describe specific embodiments and are not intended to limit the present invention. Singular forms include plural referents unless the context clearly indicates the contrary. It is to be understood that the term "comprise" or "have" as used herein is intended to indicate that there is a feature, number, step, operation, component, part, or a combination thereof described in the specification, and does not exclude the presence or the possibility of addition of one or more features, numbers, steps, operations, components, parts, or a combination thereof.

Hereinafter, preferred embodiment of the present invention will be described in detail with reference to the accompanying drawings. It should be noted that the same components are denoted by the same reference numerals in the accompanying drawings. Moreover, detailed descriptions of well-known functions and configurations that may obscure the gist of the present invention will be omitted. For the same reason, some components are exaggerated, omitted, or schematically illustrated in the accompanying drawings.

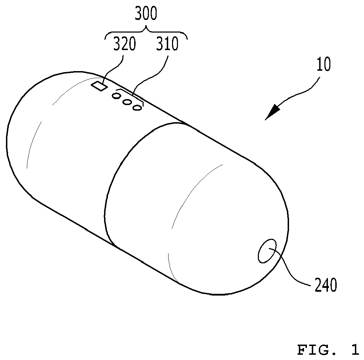

FIG. 1 is a view showing the appearance of an oral drug delivery device according to the present invention. The drug delivery device 10 of the present invention is an orally ingestible device that is introduced into the digestive tract through the mouth, and it is manufactured in a size that is not difficult to swallow, similar to the sizes of conventional oral formulations. The oral drug delivery device 10 is made of a material that is not damaged by digestive juices such as gastric acid or pancreatic juice, and it has airtightness that prevents fluids, such as digestive juices or moisture, as well as food particles from the digestive process, from penetrating into its' interior. It is exemplarily shown in FIG. 1 that electrodes of a dissolved oxygen sensor 310 or a pH sensor 320 included in a sensing unit 300 are exposed on the surface of the oral drug delivery device 10.

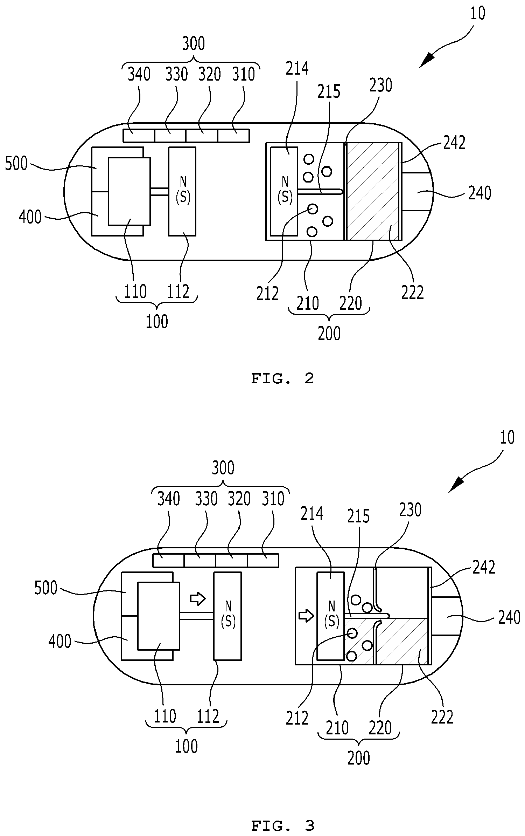

FIG. 2 is a schematic diagram showing the internal configuration of the oral drug delivery device of FIG. 1, which is in an initial inactive state before oral intake.

Referring to FIG. 2, the oral drug delivery device 10 of the present invention generally comprises a driving unit 100, a substance-containing unit 200, a sensing unit 300, and a control unit 400. Moreover, it also comprises a power supply unit 500 for supplying power to the driving unit 100 including electric/electronic devices, the sensing unit 300, and the control unit 400. The power supply unit 500 is a well-known and commonly used component, and thus its detailed description is omitted.

The driving unit 100 includes an actuator 110 and a cylinder 112 linearly moved by the actuator 110. The actuator 110 is preferably a linear motor in terms of size, but it also can be implemented with a structure including a mechanism for converting rotational motion into linear motion.

The substance-containing unit 200 includes first and second chambers 210 and 220 and two types of membranes that are ruptured as necessary to allow the substance to move therethrough. The first chamber 210 is a kind of container containing a piston 214, which linearly moves in conjunction with the cylinder 112 of the driving unit 100, and an inactive first substance 212. The second chamber 220 is a container containing a second substance 222 to be activated by reacting with the inactive first substance 212. In this case, the first chamber 210 and the second chamber 220 in their initial state are isolated from each other by a separation membrane 230, ensuring their physical and chemical separation. The other membrane is an outlet membrane 242, which closes an outlet 240 allowing either the first chamber 210 or the second chamber 220 to communicate with an external environment.

Here, it is described that the first chamber 210 contains the inactive first substance 212 and the second chamber 220 contains the second substance 222 for activation of the first substance 212; however, conversely, it is also possible to configure the first chamber 210 to contain the second substance 222 and the second chamber 220 to contain the first substance 212. However, in one embodiment to be described below, the first substance 212 is a freeze-dried strain (a strain of microorganisms having specific beneficial medicinal effects), and the second substance 222 is a culture medium. It is described as such because if a free space is created in the first chamber 210, it is easier to prevent excessive pressure from acting on the first chamber 210 when the piston 214 moves to rupture the separation membrane 230.

The sensing unit 300 includes one or more sensors capable of detecting the movement or position of the drug delivery device 10. The present invention allows the drug delivery device 10 comprising the sensing unit 300 to function as a fully self-contained oral drug delivery device 10 capable of autonomously determining whether it has reached the target organ. In other words, by incorporating the sensing unit 300, there is no longer a need to observe or control the introduction process of the drug delivery device 10 into the body using an external device, which will be described in detail in the relevant section.

The control unit 400 receives a signal provided from the sensing unit 300 and is responsible for controlling the operation of the actuator 110 based on the received signal. Since the control unit 400 is a well-known and commonly used technology, its detailed description is omitted, and the control algorithm will be mainly described in the relevant section.

As described above, the drug delivery device 10 of the present invention is characterized in that the container containing a substance is divided into the first chamber 210 and the second chamber 220 so that the first chamber 210 contains an inactive first substance 212 and the second chamber 220 contains the second substance 222 for activation of the first substance 212. This configuration offers significant advantages in many ways. Specifically, when the first substance 212 and the second substance 222 are mixed to exert a meaningful physiological or pharmacological effect, after oral intake of the oral drug delivery device 10, it allows the first substance 212 and the second substance 222 to be mixed at the optimal moment to achieve the best effect, leading to superior results, compared to the case where the first substance 212 and the second substance 222 are mixed in advance and ingested. Moreover, if the first substance 212 is a substance having a main function or vulnerable to contamination or degradation, the first substance 212 can be stored for long-term preservation (in an inactive state), and thus the expiration date of the oral drug delivery device 10 can be sufficiently secured.

Furthermore, if the first substance 212 is a strain of microorganisms having specific beneficial medicinal effects on the body, only the minimum strain can be contained in the first chamber 210 and then proliferated in the body with the second substance 222 as a culture medium, and thus it is possible to significantly reduce the required amount of strain, minimize potential side effects on the human body, and substantially reduce the production costs. For example, the oral drug delivery device 10 may be configured to contain a freeze-dried strain as the first substance 212 and a culture medium as the second substance 222.

More specifically, the first substance 212 may be a strain of freeze-dried microorganisms, and in this case, the target organs of the oral drug delivery device 10 would be the digestive tracts (e.g., the stomach, small intestine or large intestine). Examples of microorganisms that can exhibit beneficial medicinal effects on the digestive tract may include anti-Helicobacter pylori probiotics in the stomach, Lactobacillus bacteria in the small intestine, and Bifidobacterium species (abbreviated as "Bifidobacteria") in the large intestine.

However, the beneficial microorganisms mentioned above are just examples, and a wide variety of other microorganisms can be targeted. Moreover, as microbial research advances, many more microorganisms could become subjects of interest in the present invention. For example, examples of anaerobic microorganisms beneficial for the large intestine may include next-generation probiotics such as Akkermansia bacteria (effective for diabetes, obesity, and immunity), Faecalibacterium and Clostridium which produce short-chain fatty acids, and Bacteroides with immunosuppressive capabilities. Therefore, microorganisms that can exert beneficial medicinal effects, as mentioned in the present invention, should be interpreted broadly.

For living microorganisms to become activated and exert their effects in the digestive tracts, a certain minimum number of organisms are required. For example, in the case of Bifidobacteria, which functions effectively in the large intestine, over 100 million organisms are required to work effectively, and thus the minimum number of strains should be in the hundreds of thousands, and freeze-dried strains of Bifidobacterium, ranging from tens of thousands to tens of millions, are cultured to produce over 100 million organisms. The strain is in the form of freeze-dried powder, the culture medium is contained in a liquid state, and a suitable weight ratio of the strain to the culture medium is about 1:10. Moreover, aerobic strains require oxygen, while anaerobic strains can be cultured and survive only when the first chamber 210 and the second chamber 220 are in an anaerobic state. Preferably, the first chamber 210 and the second chamber 220 may be filled with nitrogen to create an anaerobic environment. For example, the first chamber 210 and the second chamber 220 are filled with the first substance 212 and the second substance 222 under anaerobic conditions with nitrogen, and to exclude germs, the second chamber 220 is filled with the culture medium and sterilized using various methods (e.g., EOS gas, electron beam, etc.) before filling the first chamber 210 with freeze-dried strains.

In this way, since the first chamber 210 and the second chamber 220 of the substance-containing unit 200 contain substances that have effects on the living body, they need to be sealed off from other components including the driving unit 100. In particular, when containing the strain and the culture medium, the need for maintaining the airtight state becomes even more critical because they are more sensitive to contamination from the external environment. This is particularly important for anaerobic strains, as the maintenance of an anaerobic state is crucial for the survival of cultured strains.

Especially, since the first chamber 210 of the substance-containing unit 200 incorporates the piston 214 that moves in conjunction with the cylinder 112 of the driving unit 100, the need for maintaining the airtightness between the driving unit 100 and the substance-containing unit 200 is also critical. For example, even if the cylinder 112 and the piston 214 are physically connected (for example, connected by a rod) to each other, a sealing means should be provided at the point of physical connection to ensure that the sealed state of the substance-containing unit 200 is maintained at all times.

More preferably, referring to FIG. 2, in order to properly maintain the sealed state of the substance-containing unit 200, the cylinder 112 and the piston 214 are physically separated from each other while each containing a magnetic element, and the poles of each magnetic element are arranged to exert a repulsive force between the cylinder 112 and the piston 214. In this way, utilizing the repulsive force of the magnetic element eliminates the need for a physical connection between the cylinder 112 and the piston 214, making it easier to maintain the sealed state.

Moreover, in such a magnetic driving structure, it is easy to create a design in which the driving unit 100 and the substance-containing unit 200 are separately manufactured and placed in separate bodies and then combined into a single body. By manufacturing the driving unit 100 and the substance-containing unit 200 separately, it becomes easier to achieve the airtightness of the substance-containing unit 200. The sensing unit 300, the control unit 400, and the power unit 500 are preferably placed together with the driving unit 100; however, due to limited space, at least some of these components may be placed on the side of the substance-containing unit 200. The combined body also needs to maintain the airtightness against the external environment, and the oral drug delivery device 10 is sterilized and then airtightly packed.

FIG. 3 is a diagram showing a state in which a separation membrane 230 is ruptured by the movement of the piston 214 in the oral drug delivery device 10 of FIG. 2. Compared to FIG. 2, in the state shown in FIG. 3, as the actuator 110 operates to cause the cylinder 112 to move toward the substance-containing unit 200, the piston 214 moves a predetermined distance in conjunction with the cylinder 112, leading to the rupture of the separation membrane 230. The separation membrane 230 may be ruptured due to the increased pressure caused by the compression of the piston 214; however, as shown, a kind of needle 215 protruding from the end of the piston 214 may also pierce and rupture the separation membrane 230. Once a rupture occurs in a certain part of the separation membrane 230, it can propagate throughout the separation membrane 230, and as the isolated state is released due to the rupture of the separation membrane 230, the first substance 212 and the second substance 222 are mixed to activate the first substance (212).

The appropriate time to rupture the separation membrane 230 is determined depending on the characteristics of the substances contained in the oral drug delivery device 10. If the best effect is obtained within a few minutes after the first substance 212 and the second substance 222 are mixed, it would be appropriate to rupture the separation membrane 230 a few minutes before the delivery (release) of the substance. On the contrary, if the first substance 212 is a freeze-dried microbial strain and the second substance 222 is a culture medium, it requires a certain time to multiply the strain to over 100 million organisms, over tenfold. For example, if the target organ of Bifidobacterium is the large intestine, it takes about 2 hours to pass through the stomach and 4 hours to pass through the small intestine. As a result, for a total of 6 hours of culturing time, it would be possible to multiply the Bifidobacterium strains to over 100 million organisms. Therefore, in this case, it would be desirable that the rupture of the separation membrane 230 occurs (immediately) before oral intake. In other words, the culture time of the microbial strain should take into account the time it takes to reach the target organ. Therefore, the separation membrane 230 should be ruptured at an appropriate time by comparing both the time it takes to reach the target organ and the time required for culture.

If the oral drug delivery device 10 is ingested after the separation membrane 230 is ruptured, the drug delivery device 10 may rotate or oscillate due to the movement of the digestive tracts (peristalsis, segmentation, etc.), and as a result of these motions, the freeze-dried microbial strain and the culture medium are evenly mixed, creating an appropriate culture environment.

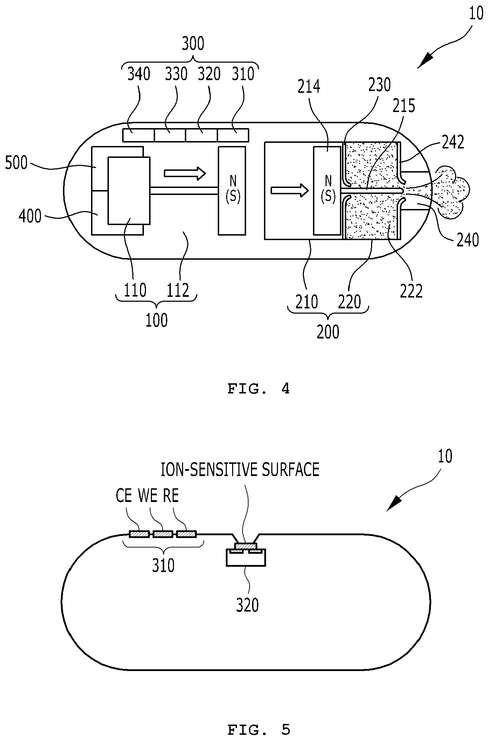

FIG. 4 is a diagram showing a state in which the piston 214 ruptures the outlet membrane 242 to release the activated substance in the oral drug delivery device 10 of FIG. 3. That is, FIG. 4 shows the state in which the first substance 212 and the second substance 222 are completely mixed and the drug delivery device 10 delivers the substances to the outside after reaching the target organ. Compared to FIG. 3, in FIG. 4, the piston 214 has moved a predetermined distance further, resulting in the rupture of the outlet membrane 242 that closes the outlet 240 provided in the second chamber 220. Due to the rupture of the outlet membrane 242, the first chamber 210 and the second chamber 220 are now in communication with the external environment. Consequently, by the movement of the piston 214 after the rupture of the outlet membrane 242, the activated first substance 212 is delivered to the target organ.

The release of substances may occur instantly or proceed slowly for an appropriate period of time. In other words, the piston 214 moves according to a predetermined release profile (time vs. stroke). In the case of Bifidobacteria, the transit time in the large intestine is typically slow, ranging from one day to a couple of days or more. Given the length of the large intestine is 180 cm, with the ascending colon occupying 25 to 30 cm, an appropriate release profile can be predetermined depending on the movement speed and distance (time) of the drug delivery device 10. Once the substance release process is completed, the oral drug delivery device 10 is expelled from the body along with feces (recovery or disposal).

In the above, it has been described that the first substance 212 and the second substance 222 are protected and contained in the oral drug delivery device 10 of the present invention, the separation membrane 230 is ruptured at an appropriate time to activate the first substance 212, and the activated first substance 212 is then released after reaching the target organ. However, another critical aspect is to recognize that the oral drug delivery device 10 has reached the target organ. Conventionally, this has been determined through two-way communication with the oral drug delivery device 10 or by observing its location using external ultrasound imaging or angiography. However, such methods cause significant inconvenience in the clinical operation of the oral drug delivery device 10. Therefore, according to the present invention, the oral drug delivery device 10 is configured as a fully self-contained oral drug delivery device 10 that, after oral intake, can autonomously determine whether it has reached the target organ and can autonomously perform all its operations.

As described above, the oral drug delivery device 10 of the present invention is provided with the sensing unit 300 comprising one or more sensors capable of detecting the movement or position thereof. Various sensors provided in the sensing unit 300 may vary depending on where they are inserted into the living body, and taking the human digestive tracts as an example, the sensing unit 300 may comprise at least one of a dissolved oxygen sensor 310, a pH sensor 320, an inertial sensor 330, and a timer 340.

The inertial sensor 330 and the timer 340 are well-known and commonly used technologies found in common household appliances such as smartphones, and thus their detailed descriptions are omitted; however, the dissolved oxygen sensor 310 and the pH sensor 320 as the electrochemical sensors will be described in more detail with reference to FIG. 5.

The dissolved oxygen sensor 310 is a sensor that measures the concentration of oxygen dissolved in a fluid. In FIG. 5, for the dissolved oxygen sensor (310), a plurality of electrodes such as a counter electrode (CE), a working electrode (WE), and a reference electrode (RE) are exposed to the surface of the drug delivery device 10. The concentration of dissolved oxygen can be measured using electrochemical techniques such as cyclic voltammetry through the plurality of electrodes.

The pH sensor 320 is a sensor that measures acidity, capable of determining acidity by measuring the concentration of hydrogen ion in a liquid. The pH values can be measured using a sensor such as an ion-sensitive field-effect transistor, which can be achieved in such a manner that a gate insulator that acts as an ion-sensitive surface of the ion-sensitive field-effect transistor is exposed to the surface of the drug delivery device 10.

The principle of distinguishing the digestive tracts using the measurement information from each sensor will be described below. The signals measured from the sensors are transferred to the control unit 400 and evaluated.

First, the dissolved oxygen sensor 310 measures the concentration of dissolved oxygen, which is the most critical factor for identifying the large intestine. The large intestine, especially the ascending colon, has an extremely low oxygen concentration value of 0, often close to 0. This makes it possible to clearly distinguish the large intestine from the stomach and the small intestine where oxygen is present.

The pH sensor 320 acquires information about acidity, which is a key factor in distinguishing between the stomach and the small intestine. The pH of the stomach is strongly acidic, ranging from 2 to 3, while the pH of the small intestine is weakly alkaline, ranging from 7 to 8 (becoming more alkaline as it moves away from the stomach), and thus it is possible to distinguish between the stomach and the small intestine based on the acidity information.

The inertial sensor 330 and the timer 340 can determine motion information such as the speed and acceleration of the oral drug delivery device 10. While it takes only a few seconds for the drug delivery device 10 to pass through the esophagus, usually 2 hours to pass through the stomach, and about 4 hours to pass through the small intestine, it takes at least a day to pass through the large intestine, and thus it is possible to obtain information for estimating the digestive tracts by analyzing the speed and acceleration of the oral drug delivery device 10. For example, the movement speed rapidly decreases in the large intestine, which becomes a critical piece of information for estimating that the drug delivery device 10 has reached the large intestine. Moreover, the motion information of the drug delivery device 10 can also be used to control the release of a substance according to a predetermined release profile for an appropriate period of time.

Furthermore, the time measured by the timer 340 also provides minimum information for determining whether the time required for the drug delivery device 10 to pass through the stomach, small intestine, and large intestine has elapsed after oral intake. That is, it takes less than 10 minutes to completely reach the stomach through the esophagus, usually 2 hours to pass through the stomach, and about 4 hours to pass through the small intestine, and thus the elapsed time information after oral intake also becomes an indicator for determining whether the device has reached the target organ.

It would be more preferable to have a plurality of various sensors. That is, it is much more accurate theoretically, probabilistically, and empirically to comprehensively determine whether the oral drug delivery device 10 containing beneficial microbial strains has reached the targeted digestive tract based on various measurement information.

For example, the control unit 400 may determine whether the device has reached the large intestine by evaluating signals from a plurality of sensors under an AND condition. For example, if both conditions, where the concentration of dissolved oxygen is 0 and it takes 6 hours after oral intake, are satisfied, it can be concluded that the device has reached the large intestine. Although the AND condition provides a stable basis for the determination, as the number of sensors increases, it would become more stringent and challenging to satisfy all conditions at the same time. Furthermore, if an error occurs in any sensor, it could permanently prevent the conditions from being satisfied, which requires an appropriate measure.

Alternatively, the control unit 400 may assign a weight to each of the signals from the plurality of sensors, quantitatively sum up the weighted sensor signals, and if the sum exceeds a predetermined reference value, determine that the device has reached the large intestine. For example, an evaluation index corresponding to each measured value, such as dissolved oxygen concentration, pH value, and movement speed, is calculated (the criteria for calculation of these evaluation indices are predefined), these evaluation indices are converted into weighted evaluation indices based on the importance of each sensor, and if the sum of these weighted evaluation indices exceeds a certain reference value, it is determined that the device has reached the target organ. This approach offers the advantages of being able to evaluate important factors more significantly, being not overly influenced by a single criterion, and making it easier to find a reasonable compromise, even if an error occurs in any sensor.

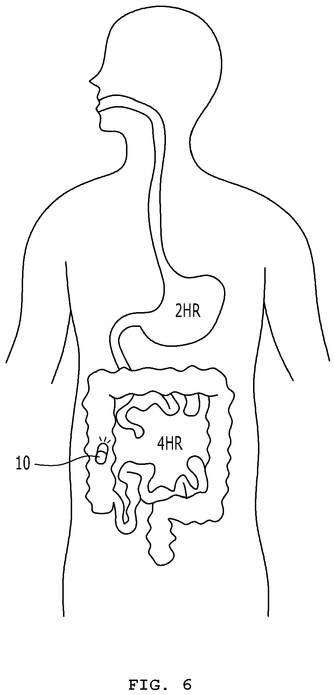

FIG. 6 is a diagram schematically showing an example of releasing a substance after the oral drug delivery device 10 reaches the ascending colon of the large intestine. Before oral intake of the oral drug delivery device 10, the separation membrane 230 is ruptured as shown in FIG, and the first substance 212 (Bifidobacterium strain) and the activated first substance 212 (culture medium) are mixed and maintained in a state suitable for cultivation. The oral drug delivery device 10 introduced into the digestive tract experiences movements such as rotation due to the movement of the digestive tracts, and as a result, the strain and the medium are vigorously mixed to activate the strain through cultivation. While moving in the intestines, the control unit 400 of the oral drug delivery device 10 analyzes the various pieces of information obtained through the sensing unit 300 to determine whether it has reached the target organ (large intestine), and if it is determined that it has reached the target organ, as shown in 4, it ruptures the outlet membrane 242 to release the activated Bifidobacteria according to a predetermined release profile. Finally, the oral drug delivery device 10 is expelled from the body along with feces, and the expelled oral drug delivery device 10 is either recovered or disposed of.

The above description is merely illustrative of the technical idea of the present invention, and various modifications and variations can be made by those skilled in the art without departing from the essential characteristics of the present invention. Therefore, the embodiments disclosed in the present invention are not intended to limit the technical idea of the present invention, but to describe the present invention, and the scope of the technical idea of the present invention is not limited by these embodiments.

### [Industrial Applicability]

The present invention provides a useful oral medical device that can effectively introduce various substances, drugs, microorganisms, etc., which can be expected to have physiological or pharmacological effects on animals including humans, into target organs in the animals' body.

## Claims

1. A swallowable oral drug delivery device, comprising:
a driving unit including an actuator and a cylinder that is linearly moved by the actuator;
a substance-containing unit including: a first chamber, which contains a piston linearly moving in conjunction with the cylinder, and an inactive first substance; a second chamber that contains a second substance for activating the first substance; a separation membrane that isolates the first chamber and the second chamber from each other; and an outlet membrane that closes an outlet allowing either the first chamber or the second chamber to communicate with an external environment;
a sensing unit that detects the movement or position of the oral drug delivery device; and
a control unit that receives a signal provided from the sensing unit and controls the operation of the actuator based on the received signal.

2. A swallowable oral drug delivery device, comprising:
a driving unit including an actuator and a cylinder that is linearly moved by the actuator;
a substance-containing unit including: a first chamber, which contains a piston linearly moving in conjunction with the cylinder, and a first chamber that contains a second substance; a second chamber that contains an inactive first substance to be activated by the second substance; a separation membrane that isolates the first chamber and the second chamber from each other; and an outlet membrane that closes an outlet allowing either the first chamber or the second chamber to communicate with an external environment;
a sensing unit that detects the movement or position of the oral drug delivery device; and
a control unit that receives a signal provided from the sensing unit and controls the operation of the actuator based on the received signal.

3. The oral drug delivery device of claim 1 or 2, wherein the substance-containing unit is sealed off from other components including the driving unit.

4. The oral drug delivery device of claim 3, wherein the cylinder and the piston are physically connected to each other, and the sealed state of the substance-containing unit is maintained at all times.

5. The oral drug delivery device of claim 3, wherein the cylinder and the piston are physically separated from each other while each containing a magnetic element, and the poles of each magnetic element are arranged to exert a repulsive force between the cylinder and the piston.

6. The oral drug delivery device of claim 1 or 2, wherein the first substance is a freeze-dried microbial strain and the second substance is a culture medium.

7. The oral drug delivery device of claim 6, wherein the first substance is anti-Helicobacter pylori probiotics, Lactobacillus bacteria or Bifidobacterium, and the target organ of the oral drug delivery device is stomach, small intestine or large intestine.

8. The oral drug delivery device of claim 1 or 2, wherein the separation membrane is ruptured as the piston moves a predetermined distance, causing the first substance and the second substance to be mixed to activate the first substance.

9. The oral drug delivery device of claim 8, wherein the first substance is a freeze-dried microbial strain and the second substance is a culture medium, and the rupture of the separation membrane occurs before oral intake of the oral drug delivery device.

10. The oral drug delivery device of claim 8, wherein after the first substance is activated and the oral drug delivery device reaches the target organ, the piston moves a predetermined distance to rupture the outlet membrane.

11. The oral drug delivery device of claim 9, wherein after the first substance is cultured and the oral drug delivery device reaches the target organ, the piston moves a predetermined distance to rupture the outlet membrane.

12. The oral drug delivery device of claim 10, wherein after the outlet membrane is ruptured, the piston moves according to a predetermined release profile.

13. The oral drug delivery device of claim 11, wherein after the outlet membrane is ruptured, the piston moves according to a predetermined release profile.

14. The oral drug delivery device of claim 1 or 2, wherein the sensing unit comprises at least one of a dissolved oxygen sensor, a pH sensor, an inertial sensor, and a timer.

15. The oral drug delivery device of claim 14, wherein the first substance is a freeze-dried microbial strain and the second substance is a culture medium, and the target organ of the oral drug delivery device is a digestive tract.

16. The oral drug delivery device of claim 15, wherein the control unit distinguishes the large intestine from the stomach and the small intestine based on the concentration of oxygen according to a signal from the dissolved oxygen sensor.

17. The oral drug delivery device of claim 15, wherein the control unit distinguishes between the stomach and the small intestine based on the acidity according to a signal from the pH sensor.

18. The oral drug delivery device of claim 15, wherein the control unit detects the speed and acceleration of the oral drug delivery device according to signals from the inertial sensor and the timer, and distinguishes between the stomach and the small intestine based on the detected speed and acceleration.

19. The oral drug delivery device of claim 15, wherein the control unit determines whether the time required for the oral drug delivery device to pass through any digestive tract has elapsed after oral intake of the oral drug delivery device according to a signal from the timer.

20. The oral drug delivery device of claim 14, wherein the sensing unit comprises a plurality of sensors selected from the dissolved oxygen sensor, the pH sensor, the inertial sensor, and the timer, and the control unit determines whether the oral drug delivery device has reached the target organ by comprehensively evaluating signals from the plurality of sensors.

21. The oral drug delivery device of claim 20, wherein the control unit determines whether the oral drug delivery device has reached the target organ by evaluating the signals from the plurality of sensors under an AND condition.

22. The oral drug delivery device of claim 20, wherein the control unit assigns a weight to each of the signals from the plurality of sensors, quantitatively sums up the weighted signals, and if the sum exceeds a predetermined reference value, determines that the oral drug delivery device has reached the large intestine.

23. The oral drug delivery device of claim 1 or 2, wherein the driving unit and the substance-containing unit are placed in separate bodies and then combined into a single body, which maintains airtightness.

24. The oral drug delivery device of claim 1 or 2, wherein at least the substance-containing unit is in an oxygen-free environment.

25. The oral drug delivery device of claim 24, wherein the substance-containing unit is in a nitrogen environment.
